# EUROPÄISCHE PATENTSCHRIFT

(11) **EP 2 271 265 B1**
(45) Veröffentlichungstag und Bekanntmachung des Hinweises auf die Patenterteilung: **29.04.2015**
(21) Anmeldenummer: 09737924.2
(22) Anmeldetag: 05.03.2009
(51) Int. Cl.: A61B 6/14, A61B 6/03

(54) **ERSTELLUNG UND PRÄSENTATION VON PATIENTENINDIVIDUELLEN PANORAMADARSTELLUNGEN**
GENERATING AND PRESENTING PATIENT-INDIVIDUAL PANORAMA IMAGES
PRODUCTION ET PRÉSENTATION DE PANORAMIQUES DENTAIRES INDIVIDUALISÉS

(30) Priorität: 02.05.2008 DE 102008021926
(43) Veröffentlichungstag der Anmeldung: 12.01.2011
(73) Patentinhaber: Sicat GmbH & CO. KG, 53177 Bonn (DE)
(72) Erfinder: BREUER, Manfred, 53347 Alfter (DE); HEY, Joachim, 53332 Bornheim (DE); HANSSEN, Nils, 53111 Bonn (DE)
(74) Vertreter: Braun-Dullaeus, Karl-Ulrich
(86) Internationale Anmeldenummer: PCT/EP2009/052642
(87) Internationale Veröffentlichungsnummer: WO 2009/132880

(56) Entgegenhaltungen:
- DE-A1-102005 055 896
- DE-T1- 10 084 386
- US-A1- 2006 275 740
- TOHNAK S ET AL: "Synthesizing panoramic radiographs by unwrapping dental CT data" ENGINEERING IN MEDICINE AND BIOLOGY SOCIETY, 2006. EMBS '06. 28TH ANNUAL INTERNATIONAL CONFERENCE OF THE IEEE, IEEE, PISCATAWAY, NJ, USA, 30. August 2006 (2006-08-30), Seiten 3329-3332, XP031390350 ISBN: 978-1-4244-0032-4

## Beschreibung

Die vorliegende Erfindung betrifft ein Verfahren zur Erstellung und Präsentation einer Panoramadarstellung eines Kieferbereiches, wobei zunächst mittels eines tomografischen Verfahrens ein Satz Volumendaten generiert wird, die den Kieferbereich eines Patienten repräsentieren, wobei die Volumendaten sich aus einer Vielzahl einzelner Volumenelemente ("Voxeln") zusammensetzen.

Solche zweidimensionalen Panoramadarstellungen von Zahnreihen und Kieferbögen sind in der Zahn- und Kleferheilkunde seit langer Zeit bekannt und beliebt, da sie dem behandelnden Arzt einen guten Überblick über die Situation geben. Besonders aussagekräftige Bilder werden entlang gekrümmter Projektionsebenen aufgenommen, deren Verlauf sich an den des Kieferknochens anpasst. Solche Panoramaaufnahmen (Orthopantomogramme) können als "klassische" Röntgenaufnahmen mit Systemen erstellt werden, bei denen eine Röntgenröhre und ein Detektor um den Kopf des Patienten herum bewegt werden.

Seit der Einführung tomografischer Aufnahmeverfahren werden solche Panoramadarstellungen auch aus Volumendaten errechnet. Dabei werden die solchermaßen errechneten Bilder so präsentiert, dass sie den gewohnten, anhand der Röntgenaufnahmen erstellten Bildern, ähneln. Diese Verfahren erzeugen somit aus dreidimensionalen Bilddaten quasi "simulierte" Orthopantomogramm-Aufnahmen, damit Zahnärzte ihre Diagnosen anhand der vertrauten Ansichten erstellen können. Bei dieser Vorgehensweise können eine Vielzahl von Möglichkeiten zur Optimierung der Bilder genutzt werden, welche die hochauflösenden Datensätze bieten. So entstehen Panoramadarstellungen mit hoher Aussagekraft.

Im Prinzip wird bei dieser Visualisierung der dreidimensionalen Bilddaten eine Projektion der Daten entlang von Projektionsstrahlen durchgeführt und das Resultat als zweidimensionales Bild auf einem Anzeigegerät dargestellt. Bei dieser Projektion können physikalische Eigenschaften, wie Emissionen oder Abschwächungen berücksichtigt werden, die den einzelnen dreidimensionalen Elementen der Bilddaten (Voxeln) anhand von Transferfunktionen zugewiesen werden. Da auch diese Darstellung letztendlich auf einer Projektion von Dichtewerten beruht, ähnelt sie zwangsläufig der oben genannten klassischen Röntgendurchleuchtung.

Ein solches Verfahren zur Erstellung von Panoramaaufnahmen aus tomografischen Daten ist beispielsweise aus der US 2006/0275740 A1 bekannt. Um bei der Berechnung der Vorzugsrichtung der anatomischen Strukturen folgen zu können, wird eine im Raum gekrümmte Abwicklungsfläche ermittelt, auf der die Projektionsstrahlen senkrecht stehen. Dadurch wird eine "Abwicklung" der Daten mit einer bestimmten Dicke erreicht. Zur Ermittlung der Abwicklungsfläche geht das Verfahren zunächst von einer initialisierenden Schnittebene aus, die bezüglich einer sitzenden Person horizontal ausgerichtet ist. In dieser Schnittebene wird eine die Kontur repräsentierende Linie innerhalb des Kieferbogens definiert, welche die Basis einer zu erstellenden Projektionsebene bildet. Ausgehend von dieser Abwicklungsfläche werden die für die Panoramadarsteilung erforderlichen Projektionen durchgeführt. Das in der der U.S 2006/0275740 A1 offenbarte Verfahren passt sich jedoch den tatsächlichen anatomischen Verhältnissen, die sich in der Vertikalen stark ändern können, nur in geringem Maße an. Zudem werden viele Gewebebereiche in die Panoramadarstellung aufgenommen, die gar nicht von Interesse sind.

Die Aufgabe der Erfindung liegt nun darin, ein gattungsgemäßes Verfahren zu schaffen, das sich einfach umsetzen lässt und das eine für den behandelnden Arzt aussagekräftige Panoramadarstellung generiert, die sich gut an die tatsächlichen anatomischen Verhältnissen anpasst.

Diese Aufgabe wird durch das Verfahren nach Anspruch 1 gelöst. Besonders vorteilhafte Ausführungsformen sind in den Unteransprüchen genannt.

Ein wesentlicher Grundgedanke der Erfindung liegt zunächst darin, nicht nur eine, sondern mehrere den Kiefer schneidende horizontale Schnittebenen festzulegen. In jeder dieser Schnittebenen lässt sich dann eine von einer inneren und einer äußeren Konturlinie begrenzte Konturfläche des Kiefers definieren. Diese Ermittlung der Konturfläche kann automatisch anhand der sich am massiven Kiefer sprunghaft ändernden Voxelwerte geschehen. Innerhalb jeder Konturflächen wird beispielsweise auf der Grundlage gewichtender Algorithmen eine Anatomiekurve definiert. Besonders erfindungswesentlich ist nun, aus diesen "schichtindividuellen" Anatomiekurven übereinanderliegender Schnittebenen eine gemeinsame Abwicklungskurve zu ermitteln, wobei dieses "Ermitteln" durch eine gewichtete rechnerische Mittelung im eigentlichen Sinne geschehen kann. Diese Abwicklungskurve definiert dann eine Abwicklungsfläche, die insbesondere vertikal im Raum steht.

Auf der Grundlage der Abwicklungsfläche werden dann wiederum für jede Schnittebene Projektionsstrahlen definiert, die senkrecht auf der Abwicklungsfläche stehen und die jeweilige in der Schnittebene liegende Konturlinie schneiden. Die Panoramaaufnahme wird nun dadurch erstellt, dass über Teilstücke der Projektionsstrahlen Integrationsintervalle definiert werden, die sich innerhalb der Konturlinie befinden. Über diese Integrationsintervalle wird dann die Information der jeweiligen Voxel integriert. Die Panoramaaufnahme besteht dann aus der Vielzahl der so ermittelten Integrale.

Der besondere Vorteil dieser Vorgehensweise ist, dass für eine Menge von Schnittebenen eine gemeinsame "beste" Abwicklungsfläche errechnet werden kann, die an die individuellen anatomischen Verhältnisse des jeweiligen Patienten optimal angepasst ist. Die Abwicklungsfläche schmiegt sich quasi tangential an die bei jedem Patienten individuell verlaufende Zahnreihe für bestimmte Schnittebenen gleichzeitig optimal an. Auf diese Weise kann beispielsweise sichergestellt werden, dass sich benachbarte Zähne der unterschiedlich geformten Unter- und Oberkiefer im Projektionsbild nicht überlagern. Ein weiterer Vorteil ist, dass auf die erfindungsgemäße Art, die einer Mittelung entsprechen kann, eine möglichst gering gewölbte Abwicklungsfläche entsteht, mit der räumliche Verzerrungen hoher Krümmung im Projektionsbild vermieden werden können. Die Gefahr, dass es, wie beim Stand der Technik möglich, wegen zu stark gekrümmter Bereiche der Abwicklungsfläche zu Überschneidungen der Projektionsstrahlen kommt, kann auf diese Weise gemindert werden. Durch das erfindungsgemäße Vorgehen kann wegen der patientenindividuell angepassten und für verschiedene Schichten unterschiedlichen Fokuskurven ein besonders hoher Kontrast in den darzustellenden anatomischen Strukturen erreicht werden.

Mit der erfindungsgemäßen Vorgehensweise werden die Mittelpunkte der einzelnen Projektionsstrahlabschnitte nicht mehr durch die Projektionsebene selber bestimmt. Stattdessen werden diese durch den Schnitt jedes (eigentlich unendlich langen) Strahls mit einer gekrümmten Fokusebene berechnet, die optimal an die darzustellende Anatomie angepasst ist.

In einer besonders vorteilhaften Ausführungsform wird der Beitrag jedes Voxels am Projektionswert durch die Reihenfolge der Voxelwerte entlang der Projektionsrichtung bestimmt, sodass von der Fokuskurve entfernte Voxel durch die zur Fokuskurve näheren Voxel semi-transparent verdeckt werden. Das hat eine zusätzliche exponentielle Gewichtung der der Fokuskurve näher gelegenen Voxel zur Folge, was zu einem erhöhten Kontrast innerhalb dieser Voxelbereiche führt. Zudem kann dann durch die semi-transparente Verdeckung, im Gegensatz zur reinen Röntgenprojektion, die räumliche Lage einzelner Strukturen in der Tiefe zueinander beurteilt werden.

Durch die "schwarnmartige", mit vielen Löchern versehene Struktur des Kieferknochens ist eine Detektierung der äußeren Knochenkanten (Konturlinien) mit einem lokalen Operator nur schwer durchführbar, schließlich würden durch den eingeschränkten "Horizont" des lokalen Operators viele der inneren, zu den Löchern gehörenden Kanten, von den äußeren Knochenkanten nicht unterscheidbar sein. Durch die Vorverarbeitung mit einem morphologischen Schließungsoperator auf den Grauwertdaten können Löcher im Knochen, die eine Mindestgröße nicht überschreiten, geschlossen werden. Durch geeignete Wahl der Mindestgröße werden nun nur noch Kanten detektiert, die (die Größe der Struktur betreffend) dominante Kanten sind. Insofern ist es vorteilhaft, jede Fokuskurve durch Vorverarbeitung mittels morphologischer Operatoren oder durch eine Kombination von Kanten und Intensitätsinformation zu ermitteln.

Auch ist es vorteilhaft, den Beitrag jedes Voxels am Projektionswert durch die Voxelwerte in einer Nachbarschaft des Voxels zu bestimmen. Dabei kann die Größe der Nachbarschaft variabel sein und kann insbesondere auch für jeden Voxel das gesamte Volumen umfassen. Dadurch kann der Kontrast von Strukturen bestimmter Form (strukturell) oder bestimmter Häufigkeit (statistisch) innerhalb der Nachbarschaft gezielt hervorgehoben werden. So können beispielsweise Nervkanäle, die einen typischen Durchmesser besitzen, besonders gut sichtbar gemacht werden (strukturell). Weiterhin kann der Kontrast von besonders häufig auftretenden Voxelwerten gemindert werden, die mit großer Wahrscheinlichkeit wenig diagnostische Information enthalten, da sie beispielsweise zum Weichgewebe gehören.

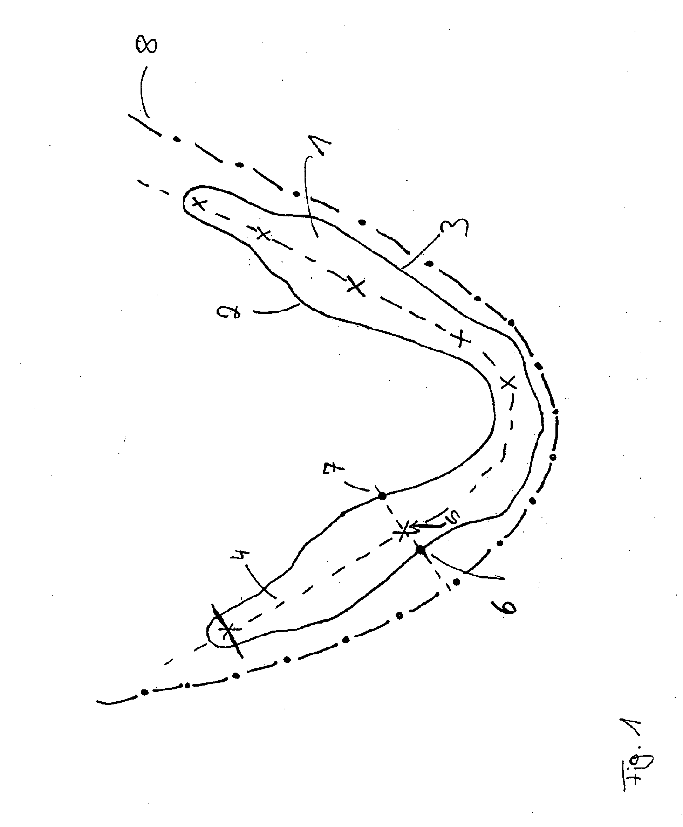

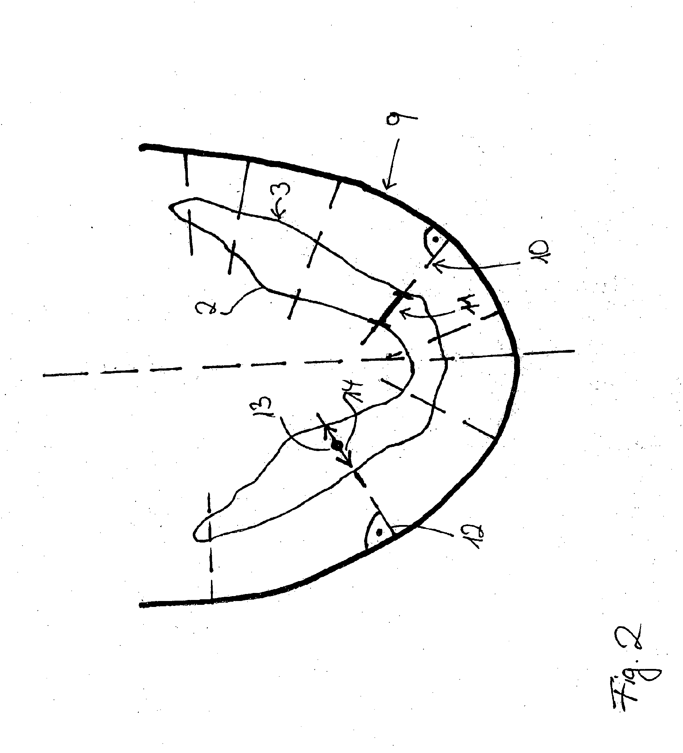

Nachfolgend wird die Erfindung anhand der Figuren 1 und 2 näher erklärt. Dabei zeigen:
- Figur 1: die Erzeugung von Fokuskurven und
- **Figur** 2: die Erzeugung von Integrationsintervallen.

Anhand der Figur 1 sind Schritte des erfindungsgemäßen Verfahrens dargestellt: Dabei wurde zunächst mittels eines tomografischen Verfahrens ein Satz Volumendaten generiert, die den Kieferbereich eines Patienten repräsentieren. Diese Volumendaten setzen sich aus einer Vielzahl einzelner Volumenelemente ("Voxel") zusammen. Innerhalb der Volumendaten werden einzelne Schnittebenen herausgegriffen, die horizontal durch den Kiefer verlaufen. Die Figur 1 zeigt eine dieser Schnittebenen, wobei jede der Schnittebenen jeweils mindestens ein Voxel stark ist. Gegebenenfalls kann jedoch über einige "Voxel"-Schichten gemittelt werden. In der Schnittebene ist die Konturfläche 1 des geschnittenen Kieferknochens zu erkennen, die von einer inneren 2 und einer äußeren Konturlinie 3 begrenzt wird. Die Konturlinien 2 und 3 können mittels entsprechender Erkennungssoftware innerhalb der Schnittebenen automatisch ermittelt werden, da sich an diesen Stellen die von den Voxeln repräsentierten Dichtewerte drastisch verändern.

Als nächster Schritt wird innerhalb einer jeden Konturfläche 1 eine Fokuskurve 4 definiert die sich als Verbindung einzelner Fokuspunkte 5 ergibt. Zur Findung der Fokuspunkte 5 können in gewissem Abstand voneinander Punkte 6 auf einer der beiden Konturlinien, hier der äußeren Konturlinie 3, definiert werden, wobei zu jedem der Punkte 6 der jeweils möglichst nahe Gegenpunkt 7 auf der anderen Konturlinie, in diesem Fall der inneren Konturlinie 2, gefunden wird. Der Mittelpunkt der die beiden Punkte 6 und 7 verbindenden Strecke kann als Fokuspunkt 5 definiert werden, wobei an dieser Stelle auch noch eine Mittelung benachbarter Punkte stattfinden kann. Durch alle Fokuspunkte 5 wird als Mittellinie die Fokuskurve 4 gelegt. Die einzelnen Fokuskurven kommen somit als Mittellinien mittig im Knochen und/oder in den Zahnreihen zu liegen. Derart wird mit allen Schnittebenen verfahren, so dass sich eine Menge übereinanderliegender Fokuskurven 4 unterschiedlicher Wölbung ergeben. In Figur 1 ist als strich punktierte Kurve 8 eine weitere Fokuskurve aus einer anderen Schnittebene eingezeichnet.

In Fällen, bei denen auf der einen Seite des Kiefers Zähne fehlen, kann der Verlauf der Fokuskurven abschnittsweise von der jeweils anderen Seite übernommen werden. Diese Interpolation kann automatisch erfolgen, da fehlende Zähne oder fehlender Knochen automatisch erkannt werden können.

Im nächsten Schritt wird aus allen Fokuskurven übereinanderliegender Schnittebenen eine gemeinsame Abwicklungskurve ermittelt, wobei diese "Ermittelung" im einfachsten Fall eine mathematische Mittelung sein kann, die gegebenenfalls gewichtet ist. Im vorliegenden Fall wird die Abwicklungskurve 9 ermittelt, die - auch wegen Gründen der Übersichtlichkeit - wiederum vor der Konturfläche 1 verläuft (siehe Figur 2). In diesem Fall wurde der geometrischen Mittelung der Fokuskurven ein Offset hinzugegeben. Durch diese Abwicklungskurve 9 wird eine nicht dargestellte Abwicklungsfläche definiert, die in diesem Fall vertikal aufgestellt ist und damit quasi als elliptisch gebogene Wand vor dem Kiefer angeordnet ist. In diesem Beispiel ist die Abwicklungskurve 9 symmetrisch zur Achse 15, so dass bei der Panoramadarstellung Vergleiche zwischen der linken und der rechten Kieferhälfte gezogen werden können.

Im weiteren Verfahren wird von der Abwicklungsfläche (hier dargestellt durch die Abwicklungskurve 9) ausgehend wiederum jede Schnittebene herangezogen, wobei Projektionsstrahlen 10 definiert werden, die in der Schnittebene senkrecht auf der Abwicklungsfläche stehen und die jeweilige Konturlinien 2 und 3 schneiden. Auf jeden der Projektionsstrahlen 10 werden nunmehr Integrationsintervalle 11 definiert, die in diesem Fall von der inneren Konturlinie 2 zur äußeren Konturlinie 3 reichen. Über diese Integrationsintervalle 11 wird die Information der Voxel integriert, wobei das Ergebnis jeder derartigen Integration jeweils ein Bildelement der Panoramadarstellung ergibt. Dabei wird der Beitrag eines jeden Voxels zum Projektionswert anhand der Voxeldaten durch ein Computerprogramm automatisch ermittelt.

In einer besonderen Ausführungsform wird anhand der zugrundeliegenden Anatomie des Kiefers auf jedem Projektionsstrahl 12 ein Referenzpunkt 13 definiert, von dem aus insbesondere zu beiden Seiten (Pfeile 14), entlang der Projektionsrichtung eine Projektion durch Integrationen ermittelt wird. Auf diese Weise kann der Kontrast optimiert werden, da wegen der minimierten Länge der Projektionsstrahlen nur ein Abschnitt des Projektionsstrahls zur Auswertung beiträgt. Weiterhin wird im Gegensatz zum Stand der Technik die Projektionsrichtung durch diese Vorgehensweise bilateral in beide Richtungen der Pfeile 14 vom Referenzpunkt 13 ausgehend durchgeführt. Dadurch liegt nun die Stelle mit dem höchsten Kontrast auf jedem Projektionsstrahl stets auf der Fokusebene.

## Patentansprüche

1. Verfahren zur Erstellung und Präsentation einer Panoramadarstellung eines Kieferbereiches, wobei zunächst mittels eines tomografischen Verfahrens ein Satz Volumendaten generiert wird, die den Kieferbereich eines Patienten repräsentieren, wobei die Volumendaten sich aus einer Vielzahl einzelner Volumenelemente ("Voxeln") zusammensetzen,
wobei mehrere den Kiefer schneidende horizontale Schnittebenen festgelegt werden,
wobei innerhalb einer jeden Schnittebene eine von einer inneren (2) und einer äußeren Konturlinie (3) begrenzte Konturfläche (1) des Kiefers definiert wird,
wobei innerhalb der Konturflächen (1) jeweils Fokuskurven (4,8) definiert werden,
wobei aus den Fokuskurven (4,8) übereinanderliegender Schnittebenen eine gemeinsame Abwicklungskurve (9) vermittelt wird, wobei die Abwicklungskurve (9) eine insbesondere vertikal aufgestellte Abwicklungsfläche definiert,
wobei ausgehend von der Abwicklungsfläche in jeder Schnittebene Projektionsstrahlen (10,12) definiert werden, die senkrecht auf der Abwicklungsfläche stehen und die jeweiligen Konturlinien (2,3) schneiden, dass auf den Projektionsstrahlen (10,12) Integrationsintervalle (11) definiert werden, über die Information der Volumenelemente integriert wird, und
wobei eine Integration jeweils ein Bildelement der Panoramadarstellung bildet,
**dadurch gekennzeichnet,**
**dass** die Integrationsintervalle innerhalb der Konturflächen (1) definiert werden.

2. Verfahren nach Anspruch 1,
**dadurch gekennzeichnet,**
**dass** anhand der zugrundeliegenden Anatomie auf jedem Projektionsstrahl (10,12) ein Referenzpunkt (13) definiert wird, von dem aus insbesondere zu beiden Seiten, entlang der Projektionsrichtung eine Projektion durch Integrationen ermittelt wird.

3. Verfahren nach Anspruch 2,
**dadurch gekennzeichnet,**
**dass** jeder Referenzpunkt (13) vom Schnittpunkt des Projektionsstrahls und der für die jeweilige Schnittebene ermittelten Fokuskurve (4,8) gebildet wird.

4. Verfahren nach einem der vorherigen Ansprüche,
**dadurch gekennzeichnet,**
**dass** die Integrationsintervalle von der inneren (2) bis zur äußeren Konturlinie (3) reichen.

5. Verfahren nach einem der vorherigen Ansprüche,
**dadurch gekennzeichnet,**
**dass** die Fokuskurven (4,8) und/oder die Abwicklungskurve (9) automatisch durch ein Computerprogramm anhand die zugrundeliegende Anatomie repräsentierenden Voxeldaten ermittelt werden.

6. Verfahren nach einem der vorherigen Ansprüche,
**dadurch gekennzeichnet,**
**dass** die Fokuskurven (4,8) jeweils als Mittellinien innerhalb der Konturflächen (1) definiert werden und somit mittig im Knochen und/oder in den Zahnreihen zu liegen kommen.

7. Verfahren nach Anspruch 6,
**dadurch gekennzeichnet,**
**dass** jede Fokuskurve (4,8) durch Vorverarbeitung mittels morphologischer Operatoren oder durch eine Kombination von Kanten und Intensitätsinformation anhand der zugrundeliegenden Anatomie ermittelt wird.

8. Verfahren nach Anspruch 1,
**dadurch gekennzeichnet,**
**dass** jede Fokuskurve (4,8) und damit auch die Abwicklungskurve (9) bezüglich des Mittelpunktes ihrer Bogenlänge spiegelsymmetrisch ist.

9. Verfahren nach Anspruch 8,
**dadurch gekennzeichnet,**
**dass** aufgrund anatomischer Verhältnisse fehlende Teile von Fokuskurven (4,8) durch die entsprechenden Teile der gegenüberliegenden Seite ersetzt werden, um fehlende Zähne und/oder fehlenden Knochen zu extrapolieren respektive zu interpolieren.

## Claims

1. Method for preparation and presentation of a panoramic visualization of a maxillary region, first of all a set of volume data that represent the maxillary region of a patient being generated by means of a tomographic method, the volume data being composed of a plurality of individual volume elements ("voxels"),
wherein several horizontal cutting planes that intersect the jaw are established,
wherein within each cutting plane, a contour surface (1) of the jaw that is bordered by an inner contour line (2) and an outer contour line (3) is defined, wherein focal curves (4, 8) are respectively defined within the contour surfaces (1),
wherein a common development curve (9) is determined from the focal curves (4, 8) of the cutting planes that lie on top of one another, the development curve (9) defining a development surface that has been set up in particular vertically,
wherein starting from the development surface, projection beams (10, 12) are defined in each cutting plane, that are perpendicular on the development surface and intersect the respective contour lines (2, 3),
that on the projection beams (10, 12) integration intervals (11) are defined, on which information of the volume elements is integrated, and
wherein one integration forms one pixel of the panoramic visualization at a time
**characterized in**
**that** the integration intervals are defined within the contour surfaces (1).

2. Method according to claim 1,
**characterized in**
**that** using the underlying anatomy on each projection beam (10, 12), one reference point (13) is defined, from which a projection is determined by integration along the projection direction, in particular to both sides.

3. Method according to claim 2,
**characterized in**
**that** each reference point (13) is formed from the intersection point of the projection beam and the focal curve (4, 8) that is determined for the respective cutting plane.

4. Method according to any of the preceding claims,
**characterized in**
**that** the integration intervals extend from the inner contour line (2) to the outer contour line (3).

5. Method according to any of the preceding claims,
**characterized in**
the focal curves (4, 8) and/or the development curve (9) are automatically determined by a computer program using voxel data that represent the underlying anatomy.

6. Method according to any of the preceding claims,
**characterized in**
the focal curves (4, 8) are each defined as centerlines within the contour surfaces (1) and thus come to rest centrically in the bone and/or in the rows of teeth.

7. Method according to claim 6,
**characterized in**
**that** each focal curve (4, 8) is determined by preprocessing by means of morphological operators or by a combination of edges and intensity information using the underlying anatomy.

8. Method according to claim 1,
**characterized in**
**that** each focal curve (4, 8) and thus also the development curve (9) is mirror-symmetrical with respect to the midpoint of its arc length.

9. Method according to claim 8,
**characterized in**
**that** parts of the focal curves (4, 8), which are missing due to anatomical conditions, are replaced by the corresponding parts of the opposite side, in order to extrapolate or interpolate missing teeth and/or missing bones.

## Revendications

1. Procédé de réalisation et de présentation d'une représentation panoramique d'une zone maxillaire, dans lequel on génère d'abord au moyen d'un procédé tomographique un ensemble de données de volume représentant la zone maxillaire d'un patient, dans lequel les données de volume se composent d'une pluralité d'éléments de volume (voxels) individuels,
dans lequel on détermine plusieurs plans d'intersection horizontaux coupant la mâchoire,
dans lequel on définit à l'intérieur de chaque plan d'intersection une surface de contour (1) de la mâchoire, délimitée par une ligne de contour intérieure (2) et une ligne de contour extérieure (3),
dans lequel on définit à l'intérieur des surfaces de contour (1) respectivement des courbes de focalisation (4, 8),
dans lequel on détermine à partir des courbes de focalisation (4, 8) de plans d'intersection superposés une courbe de développement commune (9), la courbe de développement (9) définissant une surface de développement en particulier en position verticale,
dans lequel on définit en partant de la surface de développement dans chaque plan d'intersection des faisceaux de projection (10, 12) perpendiculaires à la surface de déroulement et coupant les lignes de contour (2, 3) respectives, de sorte que sur les faisceaux de projection (10, 12), des intervalles d'intégration (11) sont définis sur lesquels les informations des éléments de volume sont intégrées, et
dans lequel une intégration constitue respectivement un élément d'image de la représentation panoramique,
**caractérisé en ce que** les intervalles d'intégration sont définis à l'intérieur des surfaces de contour (1).

2. Procédé selon la revendication 1, **caractérisé en ce qu'**à l'aide de l'anatomie sous-jacente, on définit sur chaque faisceau de projection (10, 12) un point de référence (13) à partir duquel une projection est déterminée par intégrations, en particulier vers les deux côtés suivant la direction de projection.

3. Procédé selon la revendication 2, **caractérisé en ce que** chaque point de référence (13) est formé par le point d'intersection du faisceau de projection et de la courbe de focalisation (4, 8) déterminée pour le plan d'intersection respectif.

4. Procédé selon l'une quelconque des revendications précédentes, **caractérisé en ce que** les intervalles d'intégration vont de la ligne de contour intérieure (2) jusqu'à la ligne de contour extérieure (3).

5. Procédé selon l'une quelconque des revendications précédentes, **caractérisé en ce que** les courbes de focalisation (4, 8) et/ou la courbe de développement (9) sont déterminées automatiquement par un programme informatique à l'aide de données de voxel représentant l'anatomie sous-jacente.

6. Procédé selon l'une quelconque des revendications précédentes, **caractérisé en ce que** les courbes de focalisation (4, 8) sont définies respectivement comme des lignes médianes à l'intérieur des surfaces de contour (1) et sont ainsi situées au centre de l'os et/ou des rangées de dents.

7. Procédé selon la revendication 6, **caractérisé en ce que** chaque courbe de focalisation (4, 8) est déterminée par un prétraitement au moyen d'opérateurs morphologiques ou par une combinaison d'arêtes et d'informations d'intensité à l'aide de l'anatomie sous-jacente.

8. Procédé selon la revendication 1, **caractérisé en ce que** chaque courbe de focalisation (4, 8), et donc également la courbe de développement (9), est symétrique par rapport au centre de sa longueur d'arc.

9. Procédé selon la revendication 8, caractérisé en ce des parties des courbes de focalisation (4, 8) qui manquent en raison des conditions anatomiques sont remplacées par les parties correspondantes du côté opposé pour extrapoler ou interpoler des dents manquantes et/ou de l'os manquant.
